# EUROPÄISCHE PATENTSCHRIFT

(11) **EP 2 881 031 B1**
(45) Veröffentlichungstag und Bekanntmachung des Hinweises auf die Patenterteilung: **06.03.2019**
(21) Anmeldenummer: 14194853.9
(22) Anmeldetag: 26.11.2014
(51) Int. Cl.: A61B 1/00, A61B 1/06, A61B 1/07, G02B 6/42, G02B 23/24

(54) **Endoskop, Exoskop oder Mikroskop und Verfahren zur Beleuchtung eines Betätigungsbereiches eines Endoskops, Exoskops oder Mikroskops**
Endoscope, exoscope or microscope and method for illuminating a field of operation of an endoscope, exoscope or microscope
Endoscope, exoscope ou microscope et procédé d'éclairage d'une zone de commande d'un endoscope, exoscope ou microscope

(30) Priorität: 05.12.2013 DE 102013113511
(43) Veröffentlichungstag der Anmeldung: 10.06.2015
(73) Patentinhaber: Karl Storz SE & Co. KG, 78532 Tuttlingen (DE)
(72) Erfinder: Baumann, Harald, 78532 Tuttlingen (DE)

(56) Entgegenhaltungen:
- DE-A1-102010 013 307
- DE-C1- 10 001 289
- DE-U1- 29 812 048
- JP-A- 2002 102 163
- US-A1- 2011 257 483

## Beschreibung

Die Erfindung betrifft ein Endoskop, Exoskop oder Mikroskop sowie ein Verfahren zur Bestimmung einer Zuordnungsfunktion für ein Endoskop, Exoskop oder Mikroskop.

Aus dem deutschen Patent DE 1113788 B ist ein Endoskop bekannt, das eine abgesetzte Lichtquelle zeigt, deren Licht über einen Lichtleiter in das Endoskop eingekoppelt und vom proximalen Ende des Endoskops mit dem Okular zum distalen Ende des Endoskops geleitet wird. Dabei befindet sich das Endoskop typischerweise in einer Körperhöhle und gibt dort das zugeführte Licht zur Beleuchtung der Körperhöhle in eine im Wesentlichen einzige Richtung ab. Die durch die abgesetzte Lichtquelle erzeugte erhebliche Wärme kann durch das Absetzen der Lichtquelle zu keinen Schädigungen des Patienten führen. Weiterhin erweist sich die Handhabung dieses Endoskops als angenehm, da die Abwärme der Lichtquelle nicht zu einer merklichen Erwärmung des Endoskops führt.

Aus der Offenlegungsschrift DE 10 2011 054 031 A1 ist ein Exoskop bekannt, das zum Beobachten und Beleuchten eines Objektfeldes an einem Patienten von einer Stelle abseits des Körpers des Patienten dient. Über eine abgesetzte Lichtquelle wird über ein Lichtfaserkabel das Licht zur Beleuchtung an das Exoskop zugeführt und im Exoskop mithilfe von Lichtfasern an das distale Ende mit dem Kopfteil weitergeleitet und dort zur Beleuchtung des Objektfeldes verwendet. Dabei befinden sich am Kopf zwei Austrittsöffnungen für das Licht, in die zwei Teilbündel des im Exoskop angeordneten Lichtfaserbündels münden.

Aus der DE 10 2007 063 262 A1 ist eine Beleuchtungsvorrichtung zum Erzeugen von Licht für die Endoskopie oder Mikros-kopie bekannt. Diese zeigt zur Vermeidung der Probleme der Erwärmung der Lichtquelle, die ein Array von LEDs aufweist, mehrere Kühlvorrichtungen unter Verwendung von Heat-Pipes. Diese Kühlung erweist sich als sehr aufwändig.

Endoskope mit variabler, einstellbarer Blickrichtung sind aus der EP 2 263 519 A2 und der EP 2 446 810 A1 bekannt. Diese Endoskope bedürfen einer passgenauen Beleuchtung des Bereiches, in den, abhängig von der eingestellten Blickrichtung, geblickt wird. Dabei werden zwei Möglichkeiten dargestellt. Zum einen wird großflächig der gesamte mögliche Blickbereich beleuchtet. Zum anderen wird vorgeschlagen, nicht nur die Blickrichtung selektiv zu wählen, sondern auch zusätzlich und parallel dazu die Beleuchtungsrichtung selektiv anzupassen, indem ein Lichtleiter am distalen Ende des Endoskops mit verschwenkt wird und dadurch die Beleuchtungsrichtung verändert wird. Diese Lösung erweist sich als mechanisch sehr aufwändig und benötigt im Bereich des distalen Endes ein beachtlichtes Volumen, das das Einbringen einer derartigen variablen Beleuchtung bei Endoskopen mit geringem Rohrdurchmesser erheblich einschränkt. Bei der großflächen Beleuchtung tritt das Problem der Wärmebelastung im Bereich der Lichtquelle deutlich hervor.

Weiterhin ist aus der US-Patentanmeldung US 2006/0171693 A1 ein Endoskop bekannt, das in seinem Griff mehrere voneinander getrennte Lichtquellen aufweist, die jeweils mit einem eigenen Lichtfaserbündel verbunden sind und jeweils das erzeugte Licht direkt in das jeweils zugeordnete Lichtfaserbündel einkoppeln. Jedes Lichtfaserbündel leitet anschließend das Licht an ihr distales Ende weiter. Am distalen Ende des Endoskops münden die distalen Enden der Lichtfaserbündel jeweils getrennt voneinander in voneinander abgesetzten Flächen zum Auskoppeln des Lichtes. Die Lichtfaserbündel mit den Flächen zum Auskoppeln sind gleichmäßig um den zentralen Bildübertragungskanal angeordnet und strahlen am distalen Ende parallel in dieselbe Richtung ab. Dieses Endoskop zeigt einen sehr aufwändigen Aufbau. Eine Vorrichtung gemäß dem Oberbegriff des unabhängigen Anspruchs 1 ist aus der Druckschrift US 2011/257483 A1 bekannt.

Der vorliegenden Erfindung liegt die Aufgabe zugrunde, ein Endoskop, Exoskop oder Mikroskop zu schaffen, das bei einem einfachen Aufbau die Möglichkeit schafft, selektiv einzelne Bereiche des Betätigungsfeldes des Endoskops zu beleuchten, und das dabei ein effizientes Energie- oder Wärmemanagement zeigt. Darüber hinaus liegt der Erfindung die Aufgabe zugrunde, ein Verfahren zur Bestimmung einer Zuordnungsfunktion für ein Endoskop, Exoskop oder Mikroskop anzugeben.

Diese erfindungsgemäßen Aufgaben werden durch ein Endoskop, Exoskop oder Mikroskop mit den Merkmalen des Anspruchs 1 bzw. durch ein Verfahren zur Bestimmung einer Zuordnungsfunktion mit den Merkmalen des Anspruchs 14 gelöst. Vorteilhafte Weiterbildungen sind Gegenstand der abhängigen Ansprüche.

Das erfindungsgemäße Endoskop, Exoskop oder Mikroskop zeigt ein Lichtfaserbündel zur Übertragung von Licht vom proximalen Ende zum distalen Ende, wobei am proximalen Ende ein zusammenhängendes Bündel von Lichtfasern vorliegt, während am distalen Ende des Lichtfaserbündels mehrere Teilbündel, die voneinander separiert sind, ausgebildet sind. Im äußersten Fall können einzelne Fasern bzw. distale Faserenden als Teilbündel bezeichnet werden.

Die Lichtquelle besteht aus einer Vielzahl von arrayartig angeordneten, einzeln ansteuerbaren Einzellichtquellen und ermöglicht das Einkoppeln des erzeugten Lichtes in das proximale Ende des Lichtfaserbündels. Die Steuereinheit zum Ansteuern der Lichtquelle ist erfindungsgemäß so ausgebildet, dass sie zum selektiven Ansteuern der Einzellichtquellen geeignet ist und dies in einer Weise ermöglicht, dass sie unter Verwendung einer Zuordnungsfunktion, die beispielsweise in Form einer Zuordnungstabelle hinterlegt ist, einzelne selektive Einzellichtquellen der arrayförmigen Lichtquelle aktiviert oder deaktiviert bzw. deren Helligkeit steuert. In der Zuordnungsfunktion ist die Zuordnung eines proximalen Faserendes zu einem bestimmten Teilbündel hinterlegt. Erfindungsgemäß ist es damit möglich, selektiv die proximalen Faserenden mithilfe der diesem Faserende zugeordneten Einzellichtquelle zu beleuchten, die ihr Licht in ein gewünschtes Teilbündel leiten und damit andere unerwünschte Teilbündel nicht mit Licht beaufschlagen. Dabei sind die proximalen Enden der Fasern, die einem Teilbündel zugeordnet sind, auf einer Fläche der arrayförmigen Lichtquelle so angeordnet, dass zwischen ihren proximalen Enden andere Fasern angeordnet sind, die einem anderen Teilbündel zugeordnet sind. Dies hängt mit einer arbiträren Verteilung bzw. Führung der Lichtfasern im Lichtfaserbündel zusammen. Mithilfe der Zuordnungsfunktion wird es möglich, über eine größere Fläche verteilte Einzellichtquellen des Arrays gemeinsam so zu aktivieren, dass ihr Licht in die den Einzellichtquellen zugeordnete proximale Enden der Lichtfasern einkoppeln, die mit einem oder mehreren bestimmten Teilbündeln verbunden sind, so dass eingekoppeltes Licht nur aus diesen bestimmten Teilbündeln heraustritt. Damit ist einerseits eine selektive Lichtabgabe über ausgewählte, bestimmte Teilbündel ermöglicht und andererseits eine Verteilung der Einzellichtquellen über eine größere Fläche der arrayförmigen Lichtquelle geschaffen, so dass die lokale Erwärmung im Array der Lichtquelle trotz hoher Integrationsdichte der Einzellichtquellen beschränkt ist und dadurch die Lebenszeit der Einzellichtquellen merklich erhöht ist. Die Zuordnungsfunktion repräsentiert damit einerseits eine Zuordnung der proximalen Faserenden zu einem bestimmten Teilbündel, wie auch zugleich eine Zuordnung der Einzellichtquellen der arrayförmigen Lichtquelle zu einem bestimmten Teilbündel bzw. im Extremfall einer bestimmten Einzelfaser. Ursächlich hierfür ist, dass die Einzellichtquellen spezifisch einzelnen proximalen Faserenden zugeordnet sind.

Bevorzugterweise ist dabei jede eine einzelne Einzellichtquelle einem einzelnen Faserende so zugeordnet, dass das emittierte Licht ausschließlich in dieses einkoppelt. Als Lichtquelle mit einer Vielzahl arrayförmig angeordneter Einzellichtquellen sind insbesondere solche geeignet, die in
ihrer Größe und Abstrahlrichtung das Einkoppeln in ein oder insbesondere ein einziges Lichtfaserende ermöglichen und dabei selektiv mithilfe einer entsprechenden Steuereinheit angesteuert werden können. Zeitgleich können erfindungsgemäß eine Mehrzahl an Einzellichtquellen über eine größere Fläche, die nicht notwendigerweise die vollständige Arrayfläche umfasst, verteilt so aktiviert werden, dass sie entsprechend der Zuordnungsfunktion Licht selektiv in das Lichtfaserbündel so einkoppeln, dass nur das gewünschte Teilbündel am distalen Ende Licht emittiert. Geringe Abweichungen im Prozentbereich bei der Einkopplung von Licht in andere, unerwünschte Faserenden, die mit anderen, unerwünschten Teilbündeln gekoppelt sind, sind dabei unschädlich, soweit die Anzahl der unerwünscht verwendeten Lichtfasern im geringen Prozentbereich der gewünscht verwendeten Fasern entsprechend der Zuordnungsfunktion liegt. Das durch die unerwünscht genutzten Fasern übertragene Licht führt im Betätigungsfeld zu einem geringen, typisch nicht störenden Hintergrundlicht und auch nicht zu einer beachtlichen zusätzlichen Erwärmung im Endoskop, Exoskop oder Mikroskop.

Ein Verfahren zur Beleuchtung eines Betätigungsbereiches eines Endoskops, Exoskops oder Mikroskops zeichnet sich dadurch aus, dass mittels eines Lichtfaserbündels Licht vom proximalen Ende des Endoskops zum distalen Ende übertragen wird, wobei am proximalen Ende des Lichtfaserbündels ein einziges zusammenhängendes Bündel gegeben ist, während am distalen Ende des Lichtfaserbündels mehrere, voneinander getrennte Teilbündel gegeben sind, die insbesondere in unterschiedliche Richtungen zeigen. Dies ist gerade für ein Endoskop, Exoskop oder Mikroskop mit variabler Blickrichtung von besonderem Interesse.

Die erfindungsgemäße arrayförmige Lichtquelle besteht aus einer Vielzahl von einzeln ansteuerbaren Einzellichtquellen, die selektiv aktiviert Licht in selektive, der Einzellichtquelle zugeordnete proximale Faserenden einzelner Lichtfasern einkoppeln. Durch die erfindungsgemäße Steuereinheit werden mittels einer vorgegebenen Zuordnungsfunktion, die die Zuordnung eines proximalen Faserendes des Lichtfaserbündels zu einem Teilbündel repräsentiert, die einzeln ansteuerbaren Einzellichtquellen der Lichtquelle so angesteuert, dass nur die Einzellichtquellen aktiviert werden, die einem bestimmten gewünschten Teilbündel zugeordnet sind. Dadurch gelingt es selektiv und damit abhängig von der Zuordnungsfunktion, die insbesondere in Form einer Zuordnungstabelle realisiert sein kann, die Einzellichtquellen, die über die arrayförmige Lichtquelle großflächig mit Abstand zueinander verteilt sind, zu aktivieren und dadurch eine selektive Lichtabgabe über eines oder wenige Teilbündel zu erreichen. Dies schafft ein besonders wärme- und energieeffizientes Verfahren zur Beleuchtung des Betätigungsbereiches eines Endoskops, Exoskops oder Mikroskops.

Daneben betrifft die Erfindung auch ein Verfahren zur Bestimmung einer Zuordnungsfunktion für ein Endoskop, Exoskop oder Mikroskop, wie es zuvor beschrieben ist. Dabei werden selektiv alle oder ein Teil der arrayförmig angeordneten Einzellichtquellen insbesondere sequentiell aktiviert und mithilfe wenigstens eines optischen Detektors, der beispielsweise durch eine Kamera oder einen einfachen Fotosensor oder eine Fotodiode realisiert sein kann, erfasst, welches der verschiedenen Teilbündel am distalen Ende des Lichtfaserbündels Licht emittiert. Damit wird eine Zuordnung von einzelnen Einzellichtquellen und damit von einzelnen proximalen Faserenden zu einzelnen Teilbündeln bestimmt und in einer Zuordnungsfunktion, insbesondere als Zuordnungstabelle, erfasst und gespeichert. Damit steht diese Zuordnungsfunktion, insbesondere in Form einer Zuordnungstabelle, für die Ansteuerung von Einzellichtquellen einer arrayförmigen Lichtquelle eines erfindungsgemäßen Endoskops, Exoskops oder Mikroskops zur Verfügung. Damit wird es möglich, auf Basis der Information in der Zuordnungsfunktion zielgerichtet einzelne Einzellichtquellen zu aktivieren und dadurch z.B. ein einziges Teilbündel zur Beleuchtung des Betätigungsbereiches eines Endoskops, Exoskops oder Mikroskops so zu verwenden, dass nur aus diesem Licht emittiert wird. Durch diese einfache Art der Bestimmung einer Zuordnungsfunktion kann diese verlässlich bestimmt, erfasst und gespeichert werden, so dass die vorteilhafte Realisierung der Erfindung in Form eines erfindungsgemäßen Endoskops, Exoskops oder Mikroskops bzw. eines Verfahrens zur Beleuchtung eines solchen Endoskops, Exoskops oder Mikroskops vorteilhaft ermöglicht ist.

Nach einer bevorzugten Ausbildung des erfindungsgemäßen Verfahrens wird die Identität der Lichtquelle und/oder die Position der Lichtquelle zu dem proximalen Ende des Lichtfaserbündels erfasst und in Abhängigkeit davon eine geeignete Zuordnungsfunktion aus einem der Steuereinrichtung zugeordneten Speicher ausgewählt und zur Ansteuerung der einzelnen, selektiv ansteuerbaren Einzellichtquellen der Lichtquelle bedarfsgemäß verwendet. Erfindungsgemäß werden dabei verschiedene Zuordnungsfunktionen in einem Speicher abgelegt, die unterschiedliche Situationen des erfindungsgemäßen Endoskops, Exoskops oder Mikroskops betreffen. Diese sind durch die Verwendung von unterschiedlichen Lichtquellen aber auch durch unterschiedliche Positionen oder Orientierungen der Lichtquelle gegenüber dem proximalen Ende des Faserbündels, sei es in Form einer alternativen Auswahl oder in Form einer kumulativen Realisierung, bestimmt. Je nach Auswahl und damit Identität der verwendeten Lichtquelle, die vorzugsweise als interne Lichtquelle im Handgriff oder auch als externe Lichtquelle realisiert ist und damit auf einfache Weise den unterschiedlichen Anwendungsbereichen des Endoskops, Exoskops oder Mikroskops angepasst werden kann oder auch im Rahmen einer Wartung ersetzt werden kann, gelingt es, die jeweils passende Zuordnungsfunktion, insbesondere in Form einer Zuordnungstabelle, zu verwenden. Dabei wurde zu einem früheren Zeitpunkt die passende Zuordnungsfunktion ermittelt und in einem Speicher für die Zuordnungsfunktion des Endoskops, Exoskops oder Mikroskops hinterlegt. Deiner einer externen Lichtquelle ist darauf zu achten, dass das Kabel verdrehsicher angebracht und die Ansteuerfunktion auf das jeweilige Kabel angepasst wird.

In entsprechender Weise ist es auch von Bedeutung, dass die Zuordnungsfunktion passend zur verwendeten Position der Lichtquelle zu dem proximalen Ende des Lichtfaserbündels sowohl hinsichtlich der Orientierung, der Ausrichtung oder des seitlichen Versatzes gewählt ist.

Neben der Möglichkeit, eine Vielzahl von Zuordnungsfunktionen in einem Speicher für unterschiedliche Positionen abzulegen, besteht auch die Möglichkeit nach Erfassung der Positionierung der Lichtaustrittsfläche des Lichtfaserkabels zu der proximalen Endfläche des Lichtfaserbündels aus den realtiven Positionsdaten zueinander, insbesondere aus dem Versatz, der Ausrichtung oder der Orientierung zueinander, die zugehörigen Zuordnungsfunktionen aus einer Basiszuordnungsfunktion zu berechnen und die berechnete Zuordnungsfunktion mittels der Steuereinheit zu verwenden. Dabei können die Zuordnungsfunktionen gerade im Fall eines seitlichen Versatzes, aber auch im Fall einer geänderten Orientierung oder Ausrichtung aus einer vorab bestimmten Basiszuordnungsfunktion, die der Zuordnungsfunktion an einer vorab definierten Position entspricht, nach einfachen mathematischen Zusammenhängen die für geänderte neue Position neue Zuordnungsfunktion bestimmt werden. Die Auswirkungen eines seitlichen Versatzes, einer geänderten Orientierung oder einer geänderten Ausrichtung lassen sich durch mathematische, physikalische Abbildungsmodelle darstellen. Es ist damit möglich, aus einer bestimmen Basiszuordnungsfunktion eine Vielzahl Zuordnungsfunktionen, abhängig von dem Versatz, der Orientierung bzw. der Ausrichtung zu bestimmen und diese für den Betrieb des Endoskops, Exoskops oder Mikroskops zu verwenden. Damit kann der Speichplatz beschränkt bzw. der Bestimmungsaufwand für die Zuordnungsfunktion durch Messen deutlich begrenzt werden.

Es hat sich besonders bewährt, die Lichtquelle aus einer Vielzahl von LEDs, Halbleiterlasern oder Lichtleiterfasern zu realisieren, wobei bevorzugt entweder ausschließlich LEDs, Halbleiterlaser oder Lichtleiterfasern verwendet werden. Daneben ist auch eine Kombination dieser möglichen Einzellichtquellen möglich.

Durch die neue Entwicklung in der Miniaturisierung der LEDs zeigen diese hinsichtlich ihrer lateralen Ausdehnung dieselbe Größe wie die Querschnitte der Lichtleiterfasern. Die Halbleiterlaser sind derzeit etwas größer. Durch die Verwendung von miniaturisierten LEDs oder Lichtleiterfasern wird es möglich, eine sehr kompakte kleinflächige Lichtquelle zu erzeugen, die geeignet ist, unmittelbar mit der Endfläche des Lichtfaserbündels verbunden zu werden und dabei eine gute verlässliche Zuordnung zwischen Einzellichtquelle und einem Ende einer Lichtleiterfaser zu gewährleisten. Dies ist in besonderem Maße dann gegeben, wenn die Form und/oder die Größe des Faserendes am proximalen Ende des Lichtfaserbündels der Form und/oder der Größe der zugeordneten Einzellichtquelle entspricht, insbesondere gleich ist. Gerade wenn diese gleich sowohl hinsichtlich der Form aber auch hinsichtlich der Größe gewählt ist, gelingt es, eine optimierte 1:1-Zuordnung und damit eine sehr verlässliche Zuordnungsfunktion festzulegen und im Folgenden auch für die Steuerung der Einzellichtquellen respektive des Endoskops, Exoskops oder Mikroskops zu verwenden. Alternativ zu der gleichgewählten Größe oder Form hat es sich auch bewährt, die Einzellichtquellen so zu wählen, dass sie in ihrer Größe kleiner bzw. in ihrer Form so gewählt sind, dass sie innerhalb der Fläche des Faserendes der zugeordneten Lichtfaser zu liegen kommen und damit im Wesentlichen nur in die eine zugeordnete Faser Licht emittieren. Dabei hat es sich gezeigt, dass gewisse seitliche Abstrahlungen der Einzellichtquellen in andere, benachbarte Lichtfasern unschädlich sind, solange der überwiegende Teil der emittierten Strahlung in die gewünschte Lichtfaser einkoppelt. Insbesondere hat es sich bewährt, wenn weniger als ein bestimmter Anteil, beispielsweise 20% der emittierten Strahlung in andere, nicht erwünschte Fasern einkoppelt.

Durch das Vorsehen von miniaturisierten LEDs bzw. Lichtleiterfasern lässt sich eine besonders effiziente Ankopplung der Lichtquelle an das proximale Ende des Lichtfaserbündels gewährleisten, ohne dass erhebliche Verluste oder ausgeprägte Fehleinkopplungen gegeben sind.

Es hat sich besonders bewährt, die Form und die Größe des proximalen Endes des Lichtfaserbündels so zu wählen, dass sie der Form und/oder der Größe der Lichtquelle entspricht oder gleich ist. Damit lässt sich eine verlässliche effiziente Einkopplung des Lichtes der arrayförmigen Lichtquelle in das Ende des Lichtfaserbündels gewährleisten.

Neben der besonders bevorzugten Möglichkeit, dass die Einzellichtquellen ihr emittiertes Licht direkt in eine einzige oder einzelne wenige Lichtfasern einkoppeln und dadurch mögliche Verluste gering gehalten werden, hat es sich alternativ bewährt, zwischen den Einzellichtquellen der Lichtquelle und dem proximalen Ende des Lichtfaserbündels eine oder mehrere Optiken zur fokussierten Einkopplung des emittierten Lichtes in die Fasern anzuordnen.

Vorzugsweise wird dabei eine gemeinsame Optik für die Einzellichtquellen vorgesehen, durch die es ermöglicht ist, ein großflächiges Array aus Einzellichtquellen, z.B. aus LEDs, zu realisieren und diese mithilfe der einen einzigen Optik so auf die proximale Endfläche des Lichtfaserbündels abzubilden, dass eine eindeutige Zuordnung von einer einzigen Einzellichtquelle auf ein Faserende einer einzelnen Faser gegeben ist. Durch die Verwendung gerade einer Optik wird es ermöglicht, ein größeres Array bzw. größere Einzellichtquellen verlässlich durch eine einzige einfache optische Anordnung auf eine begrenzte proximale Endfläche des Faserbündels abzubilden.

Alternativ ist es auch möglich, einzelnen Einzellichtquellen oder mehreren benachbarten Einzellichtquellen selektive Optiken so zuzuordnen, dass ihr emittiertes Licht selektiv auf eine einzige Endfläche einer Lichtfaser abgebildet wird und damit in diese eingekoppelt wird. Dies führt dazu, dass eine Vielzahl von Einzeloptiken mit sehr kleinem Durchmesser positionsgenau fixiert werden müssen, was durch die Verwendung von Chiptechnologien möglich ist. Auch die Verwendung von Komponenten, wie Mikromechanische Spiegel (MEMS, DMD), zur zielgerichteten Ablenkung des emittierten Lichtes in Richtung einer durch die Zuordnungsfunktion vorgegebenen proximalen Endfläche einer Lichtleiterfaser ist geeignet.

Durch das Vorsehen einer oder mehrere entsprechender Optiken ist es möglich, die Lichtausbeute der Lichtquelle bzw. der Einzellichtquellen zu erhöhen, indem diese großflächiger realisiert werden können und das Beabstanden der einzelnen Einzellichtquellen in dem Array vergrößert werden kann, was die Qualität insbesondere die Beständigkeit der arrayförmigen Lichtquelle verbessert.

Es hat sich als besonders vorteilhaft erwiesen, das proximale Ende des Lichtfaserbündels mit der Lichtquelle positionsfest zu verbinden. Dadurch wird sichergestellt, dass die zu der festgelegten Position geeignete Zuordnungsfunktion dauerhaft verwendet werden kann. So ist eine einfache sichere und verlässliche und damit wärme- und energieeffiziente Realisierung des Endoskop, Exoskops bzw. Mikroskops gegeben. Neben der Möglichkeit eine positionsfeste Verbindung durch Verrasten, Verschrauben, Vernieten oder Verschweißen zu schaffen, hat es sich in besonderem Maße bewährt, die Lichtquelle mit dem proximalen Ende des Lichtfaserbündels zu verkleben. Dabei wird bevorzugt ein Kleber zwischen der Lichtquelle und dem proximalen Ende des Lichtfaserbündels verwendet, der durch seine optischen Eigenschaften die Einkopplung des Lichtes der Einzellichtquelle in die zugeordneten Fasern nicht oder nur in geringem Maße negativ beeinflusst. Diese Art der positionsfesten Verbindung schafft eine dauerhafte und einfach herzustellende Verbindung. Alternativ hat sich auch das Vorsehen von lösbaren Verbindungen, die im verbundenen Zustand positionsfest sind und dadurch sich nicht in ihrer Positionierung verändern, bewährt. Dadurch ist die Möglichkeit zum Austausch der Lichtquelle bzw. des Lichtfaserbündels, was im Rahmen von Serviceleistungen erfolgen kann, gegeben.

Eine besonders bevorzugte Ausbildung des erfindungsgemäßen Endoskops, Exoskops oder Mikroskops zeichnet sich dadurch aus, dass die Lichtquelle im Endoskop, insbesondere in dessen Griff integriert angeordnet ist und dadurch eine aufwändige Verkabelung zur Zuführung von Licht zur Beleuchtung des Betätigungsbereiches nicht notwendig ist. Dies wird insbesondere dadurch ermöglicht, dass die arrayförmige Lichtquelle vorzugsweise so klein gewählt ist, dass sie in ihrer Größe und Form der proximalen Endfläche des Lichtfaserbündels entspricht und nur mit geringem Abstand zu dieser positioniert ist. Durch diese bevorzugte Ausbildung und Positionierung der Lichtquelle zum Lichtfaserbündel ist eine sehr kompakte Anordnung geschaffen, die verlässlich z.B. im Griff oder in einem anderen Gehäusebereich des Endoskops, Exoskops oder Mikroskops angeordnet werden kann. Gerade durch die erfindungsgemäße großflächige Verteilung der aktivierten Einzellichtquellen zur Ansteuerung eines einzelnen Teilbündels gelingt es, die Wärmebelastung dieser sehr kompakten Lichtquelle zu begrenzen und z.B. in einen Griff eines Endoskops oder Exoskops zu integrieren, ohne dass dieser unangenehm erwärmt wird.

Alternativ dazu hat es sich auch bewährt, die Lichtquelle vom restlichen Endoskop, Exoskop oder Mikroskop abgesetzt und damit getrennt von diesen zu realisieren und das Licht der Lichtquelle über ein Lichtfaserkabel von der Lichtquelle zum restlichen Endoskop zu führen und dort in die proximale Endfläche des Lichtfaserbündels einzukoppeln. Diese Anordnung ermöglicht das einfache Austauschen der externen Lichtquelle, z.B. abhängig von dem Anwendungsgebiet des Endoskops, Exoskops oder Mikroskops bzw. im Fall einer Beschädigung der Lichtquelle. Die Zuordnungsfunktion ist dabei so gewählt, dass sie dieses zwischengeschaltete Lichtfaserkabel berücksichtigt. Hier ist für die Zuordnungsfunktion insbesondere von Bedeutung, ob und wie das Lichtfaserkabel die Position der Lichtfasern, in die das Licht einer Einzellichtquelle eingekoppelt wird, relativ zur Position beim Auskoppeln verändert. Diese Veränderung ist in der Zuordnungsfunktion zu berücksichtigen. Dies wird insbesondere dadurch erreicht, dass die externe Lichtquelle mit dem Lichtfaserkabel als eine Gesamtlichtquelle bei der Bestimmung der Zuordnungsfunktion betrachtet wird. Es wird also die Einheit aus Lichtquelle und Lichtfaserkabel funktionell als eine Lichtquelle betrachtet, die an der Kopplungsfläche des Lichtfaserkabels zum Endoskop und damit zu dem proximalen Ende des Lichtfaserbündels das Licht emittiert. Bei diesen Kopplungen sind Mittel vorgesehen, die eine eindeutige Positionierung, definiert festlegen, so dass insbesondere zwangsgeführt immer die gewünschte festgelegte Positionierung beim Zusammenbauen gegeben ist. Die vorgesehene eindeutige Positionierung zeichnet sich dabei insbesondere durch eine korrekte laterale Positionierung der Lichtaustrittsfläche des Lichtfaserkabels zu der proximalen Endfläche des Lichtfaserbündels, durch eine korrekte relative Orientierung (relative Drehposition) bzw. die korrekte Ausrichtung (korrekte Neigung der Flächen zueinander) aus. Dadurch ist eine verlässliche Auswahl der Zuordnungsfunktion für die Ansteuerung der Einzellichtquellen gegeben.

Alternativ hat es sich auch bewährt, die Positionierung und kumulativ oder alternativ hierzu die verwendete Lichtquelle vom erfindungsgemäßen Endoskop, Exoskop oder Mikroskop zu erfassen und abhängig davon die geeignete Zuordnungsfunktion für die Steuerung der Lichtquelle zu wählen. Für die Identifizierung der Lichtquelle hat es sich besonders bewährt, Codierungen der Lichtquelle mittels optischer und/oder elektrischer, insbesondere elektromagnetischer Codes vorzusehen und zu verwenden. Die Positionierung der Lichtquelle kann z.B. mithilfe von vorgegebenen Markern, insbesondere optischen Markern, verlässlich erfasst werden und bei der Bestimmung der Zuordnungsfunktion berücksichtigt werden. Als bevorzugte Mittel zur eindeutigen Festlegung der Position der Lichtquelle gegenüber dem proximalen Ende des Lichtfaserbündels haben sich insbesondere Anlaufkanten besonders bewährt, die eine Zwangsführung in eine vorgegebene Position der Lichtquelle gegenüber dem proximalen Ende des Lichtfaserbündels gewährleisten. Dies schafft eine sehr einfache und verlässliche Handhabung des erfindungsgemäßen Endoskops, Exoskops oder Mikroskops.

Gerade bei einem Endoskop, Exoskop oder Mikroskop mit einer variablen Blickrichtung hat es sich besonders bewährt, dass die Teilbündel am distalen Ende des Endoskops, Exoskops bzw. Mikroskops in unterschiedliche Richtungen in den potentiellen Betätigungsbereich weisen. Durch die Zuordnungsfunktion ist die Möglichkeit geschaffenen, selektiv die verschiedenen Teilbündel zu verwenden. Damit ist gerade bei diesen medizinischen Instrumenten die Möglichkeit geschaffen, sehr energieeffizient und mit einem guten Wärmemanagement eine ausreichende und sichere Beleuchtung in der gewünschten Blickrichtung des Endoskops, Exoskops oder Mikroskops zu ermöglichen und dadurch eine sichere Handhabung zu gewährleisten.

Weiterhin zeigt die Steuereinheit zum Ansteuern der Lichtquelle eines bevorzugtes erfindungsgemäßes Endoskop, Exoskop oder Mikroskop die Möglichkeit, die Lichtquelle mit einem vorgegebenen Beleuchtungsprofil so anzusteuern, dass ausgewählte Bereiche des möglichen Ausleuchtungsbereiches in vorgegebener Stärke beleuchtet werden. Dabei werden einzelne Bereiche bzw. zugeordnete Einzellichtquellen nicht aktiviert, andere gedimmt aktiviert und andere mit voller Leistung aktiviert. Dies erweitert den Einsatzbereich des erfindungsgemäßen Endoskops, Exoskops oder Mikroskops merklich.

Mit anderen Worten lässt sich die Erfindung damit beschreiben, dass in einem Endoskop, Exoskop oder Mikroskop ein unsortiertes Faserbündel zur Übertragung von Licht an eine Mehrfachlichtquelle, die als ein Array ausgebildet ist, angekoppelt wird, wobei mithilfe einer elektronischen Steuerung einzelne Fasern unterschiedlicher Teilbündel durch das Ansteuern einzelner, selektiver Einzellichtquellen der Mehrfachlichtquelle möglichst selektiv beleuchtet werden, um nur Licht für einen begrenzten Ausleuchtungsbereich zu erzeugen, der aktuell beobachtet wird.

Die erfindungsgemäße Ansteuerung der Einzellichtquellen wird bei der Montage mittels einer Vorrichtung zur Bestimmung einer Zuordnungsfunktion zwischen Teilbündeln und Einzellichtquelle erfasst und abgespeichert. Diese Zuordnungsfunktion entspricht der Zuordnung der einzelnen Teilbündel zu den proximalen Endflächen der Fasern, die den jeweiligen Einzellichtquellen zugeordnet sind.

Die Mehrfachlichtquelle zeigt eine sehr hohe Integrationsdichte der Einzellichtquellen. Die Einzellichtquellen sind bevorzugt auf demselben Substrat mit einem minimierten Trennsteg aufgebracht. Die Mehrfachlichtquelle wird vergleichbar einem CMOS-Sensor gestaltet, bei dem jedes Pixel eine eigene, separat ansteuerbare Einzellichtquelle, z.B. in Form einer LED ist. Auf dem Substrat der Mehrfachlichtquelle wird bevorzugt auch die Steuerung zur selektiven Ansteuerung der Einzellichtquellen abhängig von der ermittelten Zuordnungsfunktion angeordnet.

Diese Erfindung erweist sich als sehr kompakt, energie- und wärmeeffizient sowie als einfach herzustellen.

Im Folgenden wird die Erfindung anhand einzelner Beispiele in den Figuren beschrieben. Die Erfindung ist nicht auf diese Beispiele beschränkt.

Es zeigen:
- Fig. 1: eine schematische Darstellung eines beispielhaften distalen Endes eines Endoskops mit variabler Blickrichtung,
- Fig. 2: eine schematische Darstellung einer beispielhaften Faseransteuerung eines beispielhaften Endoskops,
- Fig. 3: eine beispielhafte Zuordnungstabelle und
- Fig. 4: eine schematische Anordnung zur Bestimmung einer Zuordnungsfunktion.

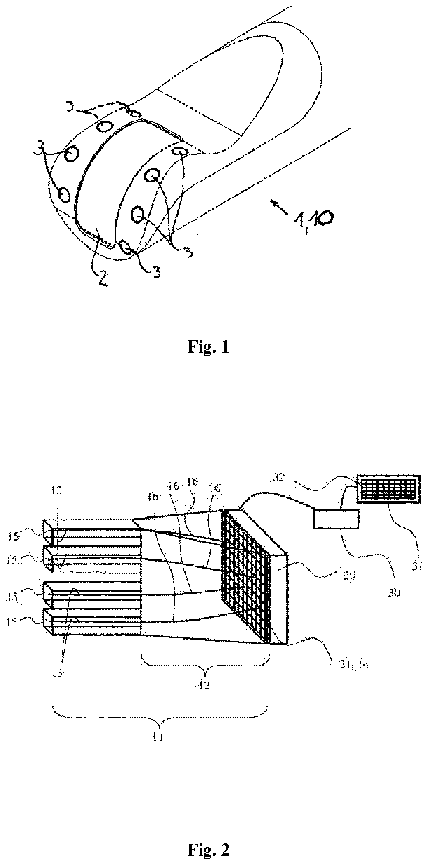

Fig. 1 zeigt in einer Schrägansicht den schematischen Aufbau eines beispielhaften distalen Endes 1 eines erfindungsgemäßen Endoskops 10. Zentral ist das Beobachtungsfenster 2, das langgestreckt ausgebildet ist und zentral im Bereich des distalen Endes 1 angeordnet ist, zu erkennen. Das Beobachtungsfenster 2 ist dabei gewölbt ausgebildet und zeigt dabei eine im Wesentlichen rechteckige Gestalt, welche im Wesentlichen zylindrisch gewölbt ausgeführt ist. Entlang der Längsausdehnung des Beobachtungsfensters 2 sind Lichtaustrittsöffhungen 3 angeordnet. Es handelt sich hier um insgesamt acht Lichtaustrittsöffnungen 3, in denen jeweils ein Teilbündel 13 des Lichtfaserbündels 11 zur Übertragung von Licht vom proximalen Ende 2 zum distalen Ende 1 des Endoskops 10 mündet. Die Teilbündel 13 sind am distalen Ende 1 in den Lichtaustrittsöffnungen 3 so miteinander verklebt, dass die Lichtaustrittsflächen gas- und flüssigkeitsdicht und damit autoklavierbar ausgebildet sind. Dabei zeigen die Teilbündel 13 der Lichtaustrittsöffnungen 3 in unterschiedliche Richtungen in den Betätigungsbereich des Endoskops 10. Mithin ist durch die verschiedenen Lichtaustrittsöffnungen 3 die Möglichkeit geschaffen, unterschiedliche Raumbereiche im Betätigungsbereich des Endoskops 10 zu beleuchten.

In Fig. 2 ist ein schematischer Aufbau des Endoskops 10 und hier des Lichtfaserbündels 11, der Lichtquelle 20, der Steuereinheit 30 mit zugeordnetem Speicher 32 dargestellt.

Die Lichtquelle 20 stellt eine flächige, rechteckige, arrayförmige Anordnung von Einzellichtquellen 21 dar. Die Einzellichtquellen 21 sind schachbrettartig in Spalten und Zeilen angeordnet und bilden die Lichtquelle 20. Die Einzellichtquellen 21 sind als superminiaturisierte LEDs auf einem gemeinsamen Substrat realisiert. Ihre Größe entspricht dem Durchmesser einer typischen Lichtfaser zur Übertragung von Licht. Jede Einzellichtquelle 21 kann durch eine
Benennung der Spalte und der Zeile eindeutig definiert werden. Die Steuereinheit 30 ist so ausgebildet, dass sie jede einzelne Einzellichtquelle 21 der Lichtquelle 20 selektiv einzeln so ansteuern kann, dass sie aktiviert wird und Licht aussendet oder deaktiviert wird.

Das erfindungsgemäße Endoskop 10 zeigt neben der Lichtquelle 20 und der Steuereinheit 30 ein Lichtfaserbündel 11, das an seinem proximalen Ende 14 ein einziges zusammenhängendes Bündel 12 aus Lichtfasern 16 zeigt. An dem anderen distalen Ende 15 des Lichtfaserbündels 11 sind vier voneinander getrennte Teilbündel aus unterschiedlichen Lichtfasern 16 zu erkennen. Die Zuordnung der einzelnen Lichtfasern 16 vom proximalen Ende 14 zu dem distalen Ende 15 ist arbiträr, das bedeutet, dass die Fasern sich ungeordnet vom proximalen Ende 14 zum distalen Ende 15 erstrecken. Damit bilden Lichtfasern 16, die ein gemeinsames Teilbündel 13 bilden, an dem proximalen Ende 14 des Lichtfaserbündels 11 keine zusammenhängende kompakte und wohlsortierte Anordnung aus Lichtfasern, sondern sind erfindungsgemäß über die Endfläche des proximalen Endes 14 des Lichtfaserbündels 11 verteilt angeordnet. Damit sind zwischen den Lichtfaserenden des einen gemeinsamen Teilbündels 13 unterschiedlich viele andere Lichtfasern 16, die zu anderen Teilbündeln 13 gehören, in der Endfläche angeordnet.

Dem proximalen Ende 14 des Lichtfaserbündels 11 ist unmittelbar benachbart die arrayförmige Lichtquelle 20 aus einer Vielzahl von Einzellichtquellen 21 angeordnet. Die Einzellichtquellen 21 sind in ihrem Querschnitt so gewählt, dass ihre Abstrahlcharakteristik mit der Endfläche einer einzelnen Lichtfaser 16 am proximalen Ende 14 des Lichtfaserbündels 11 so korreliert, dass das emittierte Licht der Einzellichtquelle 21 weitgehend oder vollständig in das proximale Ende 14 der Lichtfaser 16 einkoppelt. Es besteht eine 1:1-Zuordnung zwischen Einzellichtquelle 21 und Lichtfaser 16. Damit kann durch selektive Ansteuerung der Lichtquelle 20 mithilfe der Steueranordnung 30 eine vorgegebene Auswahl an Einzellichtquellen 21 aktiviert werden und mithilfe dieser Licht in zugeordnete einzelne proximale Enden 14 der Lichtfasern 16 eingekoppelt werden, die anschließend das Licht an ihr distales Ende 15 weiterleiten, um es dort abzugeben.

Erfindungsgemäß wird die Auswahl der aktivierten Einzellichtquellen 21 mithilfe der Steueranordnung 30 vorgenommen, die auf einen Speicher 32 mit darin abgelegten Zuordnungsfunktionen zugreift. Die Zuordnungsfunktion ist als Zuordnungstabelle 31 realisiert.

Soll Licht von dem Endoskop 10 in eine vorgegebene Richtung selektiv gestrahlt werden, so wird die Steueranordnung 30 die Einzellichtquellen 21 so selektiv ansteuern, dass nur diese Licht in die ihnen zugeordneten proximalen Enden 14 der Lichtleitfasern 16 einkoppeln, die Licht in das Teilbündel 13 leiten, das in die vorgesehene, gewünschte Richtung zeigt. Andere Teilbündel werden nicht oder nur in ungeordnetem Umfang beleuchtet. In der Zuordnungstabelle 31 des Speichers 32 ist festgelegt, welche Einzellichtquellen 21 welches Teilbündel 13 repräsentieren, so dass mithilfe der Steueranordnung 30 die Möglichkeit geschaffen ist, einzelne Teilbündel 13 selektiv mit Licht zu beaufschlagen und dementsprechend Licht in die entsprechende Abstrahlrichtung auszustrahlen.

Durch die arbiträre Verteilung der Lichtfasern 16 in dem Lichtfaserbündel 11 und damit die arbiträre Verteilung der einem Teilbündel 13 zugeordneten proximalen Enden 14 der Lichtfasern 16 in der proximalen Endfläche, die die gleiche Verteilung der zu aktivierenden Einzellichtquellen 21 in der arrayartigen Lichtquelle 20 zeigt, gelingt es, die aktivierten Einzellichtquellen 21 über eine größere Fläche zu verteilen und dadurch die Anfälligkeit gegen Überhitzung, die einen Ausfall der Einzellichtquellen 21 bewirken kann, merklich einzuschränken.

Zusätzlich kann durch die Verwendung der arbiträr verteilten Lichtfasern 16 in dem Lichtfaserbündel 11 die Fertigung der Lichtübertragungskomponenten eines Endoskops 10 erheblich vereinfacht werden, wobei dies jedoch mit einem zusätzlichen notwendigen, erfinderischen Schritt zur Ermittlung der Zuordnungsfunktion bzw. Zuordnungstabelle 31 einhergeht bzw. ein solcher Schritt erforderlich ist. Durch den teilweisen Wegfall des Sortierens und Sortierthaltens der Lichtfasern 16 des Lichtfaserbündels 11 gegenüber dem Stand der Technik kann die Fertigung merklich vereinfacht werden.

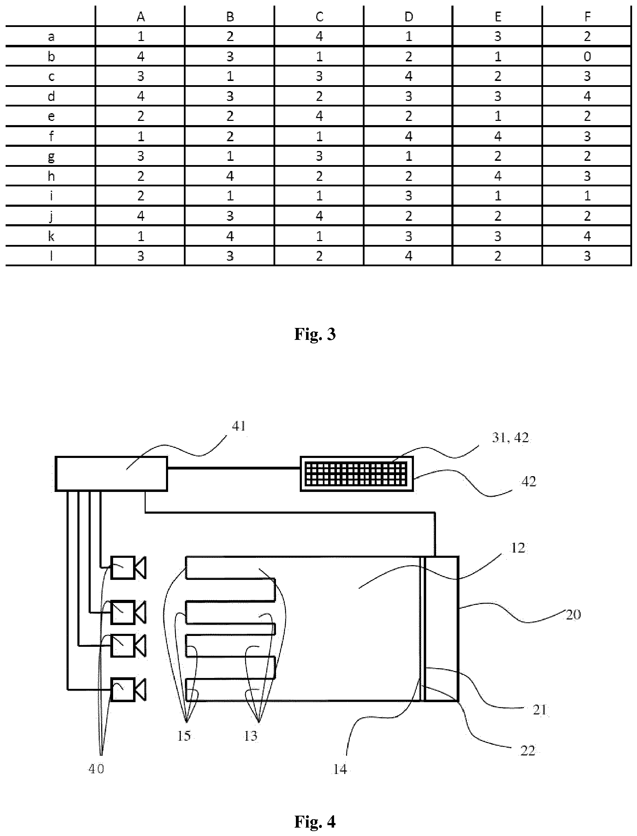

Eine beispielhafte vereinfachte Zuordnungstabelle 31 ist in Fig. 3 dargestellt. Jeder Einzellichtquelle 21 der arrayförmigen Lichtquelle 20 ist ein Element der Tabelle zugeordnet, wobei die Spalten A, B, C, D, ... und Zeilen a, b, c, d, ... der Anordnung der Einzellichtquellen 21 in der arrayförmigen Lichtquelle 20 auf dem gemeinsamen Substrat entsprechen. Der Wert in einem Feld der Zuordnungstabelle 31 entspricht einer eindeutigen Nummerierung der Teilbündel 13, die am distalen Ende 1 des Endoskops 10 münden und in unterschiedliche Richtungen des Betätigungsbereiches gerichtet leuchten können.

In dem Beispiel der Fig. 2 sind es vier Teilbündel 13. Entsprechend zeigt die vereinfachte Zuordnungstabelle 31 der Fig. 3 Felder mit den Nummern 1, 2, 3, 4, die den vier Teilbündeln 13 aus Fig. 2 entsprechen. Soll in Richtung des Teilbündels 13 mit der Nummer 1 Licht ausgestrahlt werden, so werden in der Zuordnungstabelle 31 im Speicher 32 die Felder der Zuordnungstabelle 31 selektiert, die mit dem Eintrag 1 versehen sind. Die diesen Feldern entsprechenden Einzellichtquellen 21 werden dann mittels der Steueranordnung 30 aktiviert. Andere Lichtquellen 21 werden nicht aktiviert, so dass eine über eine größere Fläche verteilte Menge an Einzellichtquellen 21 aktiviert wird und das emittierte Licht in die unmittelbar benachbarten Endflächen 14 der zugeordneten Lichtfasern 16 einkoppelt und trotz der arbiträr verteilten Lichtfasern 16 unter erfindungsgemäßer Nutzung der selektiven Ansteuerung anhand der Zuordnungstabelle 31 Licht nur aus dem Teilbündel 13 mit der Nummer 1 austritt. Mögliches Streulicht, das in andere Fasern einkoppelt, führt typischerweise nur zu einer diffusen Hintergrundbeleuchtung des Betätigungsbereiches des Endoskops 10, die praktisch unschädlich ist.

Dabei ist die arrayförmige Lichtquelle 20 so mit dem proximalen Ende 14 des Lichtfaserbündels 11 verbunden, dass diese positionsfest miteinander verklebt sind und dadurch die Zuordnung der Einzellichtquellen 21 zu den einzelnen proximalen Enden 14 der Lichtleitfasern 16 dauerhaft und eindeutig gegeben ist. Auf Basis dieser positionsfesten Zuordnung wird die Zuordnungstabelle 31 in dem Speicher 32 durch eine automatisierte Bestimmung der Zuordnungsfunktion 31 mithilfe einer entsprechenden Vorrichtung sehr verlässlich und schnell realisiert. Ein Beispiel für eine solche Anordnung zur Bestimmung einer Zuordnungstabelle 31 ist in Fig. 4 schematisch dargestellt.

Die arrayförmige Lichtquelle 20 ist mit dem Lichtfaserbündel 11 vollflächig mithilfe eines optisch transparenten Klebeelementes 22 so verbunden, dass das emittierte Licht der Einzellichtquellen 21 der Lichtquelle 20 direkt in die proximale Endfläche 14 des zusammenhängendes Bündels 12 des Lichtfaserbündels 11 eingekoppelt wird. Das verklebte Modul stellt ein vorgefertigtes Lichtübertragungsmodul dar, das in dem vormontierten Zustand in ein erfindungsgemäßes Endoskop 10 eingebaut wird. Die Ankopplung des Lichtfaserbündels 11 an die Lichtquelle 20 ist so erfolgt, dass eine Einzellichtquelle 21 Licht ausschließlich oder weitgehend ausschließlich nur in eine einzelne Lichtfaser 16 einkoppelt. Diese Lichtfaser 16 überträgt das eingekoppelte Licht vom proximalen Ende 14 zum distalen Ende 15 der Lichtleitfaser 16. Dort tritt das Licht aus der Endfläche aus und kann mithilfe von Fotodioden 40, die jeweils auf ein distales Ende eines Teilbündels 13 des Lichtfaserbündels 11 gerichtet sind, erfasst werden. Jede Fotodiode 40 ist einem einzigen Teilbündel 13 zugeordnet.

Um die Zuordnungsfunktion respektive die Zuordnungstabelle zu ermitteln, wird sequenziell Einzellichtquelle 21 für Einzellichtquelle 21 aktiviert und jeweils bestimmt, welcher Fotosensor einen Helligkeitsanstieg detektiert, so dass die Zuordnung einer bestimmten Einzellichtquelle 21 zu einer Lichtfaser 16 und damit zu einem Teilbündel 13 bestimmt und erfasst und in einem Speicher, wie z.B. der Speicher 42, gespeichert wird. Durch das sequentielle Ansteuern aller Einzellichtquellen 21 der arrayförmigen Lichtquelle 20 durch die Steuerung 41 kann die Zuordnungsfunktion, die als Zuordnungstabelle 43, 31 ausgebildet ist, für das gesamte Array aus Einzellichtquellen bestimmt und abgespeichert werden. Dabei wird in die Zuordnungstabelle 43, 31 jeweils eine Kennzeichnung des jeweiligen Teilbündels 13, beispielsweise in Form einer Nummerierung 1, 2, 3, 4 verwendet. Wird keine Erhöhung der Helligkeit bei der Aktivierung einer Einzellichtquelle 21 der arrayförmigen Lichtquelle 20 durch eine der Fotodioden 40 sensiert, so wird ein Eintrag, der einen Defekt repräsentiert, z.B. 0, eingetragen. Ein Defekt kann beispielsweise durch eine defekte Einzellichtquelle 21, einen Bruch der Lichtleitfaser oder durch eine Verschmutzung zum Beispiel im Bereich der Verklebung 22 begründet sein. Erfindungsgemäß wird die Einzellichtquelle 21, die einer Zelle der Zuordnungstabelle 31 mit dem Wert 0 zugeordnet ist, im Betrieb des Endoskops 10 durch die Steueranordnung 30 nicht angesteuert, so dass von dieser Einzellichtquelle 21 auch keine thermische oder energetische Belastung des Systems ausgeht.

Mithilfe des erfindungsgemäßen Verfahrens zur Bestimmung einer Zuordnungsfunktion für ein solches erfindungsgemäß beschriebenes Endoskop ist eine sehr verlässliche und sichere und einfache Bestimmung der Zuordnungsfunktion gegeben, die einen sehr effizienten und verlässlichen Betrieb eines erfindungsgemäßen Endoskops ermöglicht.

### Bezugszeichenliste:

- 1.: distales Ende des Endoskops
- 2.: Beobachtungsfenster
- 3.: Lichtaustrittsöffnungen
- 10.: Endoskop
- 11.: Lichtfaserbündel
- 12.: zusammenhängendes Bündel
- 13.: Teilbündel
- 14.: proximales Ende des Lichtfaserbündels bzw. der Lichtfasern
- 15.: distales Ende des Lichtfaserbündels bzw. der Lichtfasern
- 16.: Lichtfaser
- 20.: Lichtquelle
- 21.: Einzellichtquelle
- 22.: Verklebung
- 30.: Steueranordnung
- 31.: Zuordnungsfunktion, Zuordnungstabelle
- 32.: Speicher
- 40.: optischer Detektor, Photodiode
- 41.: Steuerung
- 42.: Speicher
- 43.: Zuordnungstabelle

## Patentansprüche

1. Endoskop (10), Exoskop oder Mikroskop,
mit einem Lichtfaserbündel (11) zur Übertragung von Licht von seinem proximalen Ende (14) zum seinem distalen Ende (15), das am proximalen Ende (14) ein zusammenhängendes Bündel (12) zeigt und das am distalen Ende (15) mehrere Teilbündel (13) zeigt,
mit einer Lichtquelle (20) zur Einkopplung von Licht in das eine proximale Ende (14) des Lichtfaserbündels (11) mit einer Vielzahl an arrayartig angeordneten Einzellichtquellen (21) und mit einer Steuereinheit (30) zum Ansteuern der Lichtquelle (20), wobei die Lichtquelle (20) mit einer Vielzahl an einzeln ansteuerbaren Einzellichtquellen (21) versehen ist, **dadurch gekennzeichnet dass** die Steuereinheit (30) zum Ansteuern der Einzellichtquellen (21) unter Verwendung einer Zuordnungsfunktion (31), die die Zuordnung eines proximalen Faserendes (14) zu einem Teilbündel (13) repräsentiert, geeignet ist und dass die proximalen Enden (14) der Fasern (16), die einem Teilbündel (13) zugeordnet sind, auf einer Fläche so angeordnet sind, dass zwischen ihnen proximale Enden (14) von Fasern (16), die einem anderen Teilbündel (13) zugeordnet sind, angeordnet sind, dass die Steuereinheit mittels einer vorgegebenen Zuordnungsfunktion, die die Zuordnung eines proximalen Faserendes des Lichtfaserbündels zu einem Teilbündel repräsentiert und die einzeln ansteuerbaren Einzellichtquellen der Lichtquelle so ansteuert, dass nur die Einzellichtquellen aktiviert werden, die einem bestimmten gewünschten Teilbündel zugeordnet sind.

2. Endoskop, Exoskop oder Mikroskop nach Anspruch 1,
**dadurch gekennzeichnet**, dassdie Lichtquelle (20) ein Array aus LEDs, Halbleiterlasern, Lichtleiterfaserenden aufweist.

3. Endoskop, Exoskop oder Mikroskop nach Anspruch 1 oder 2,
**dadurch gekennzeichnet**, dassdie Form und/oder die Größe des proximalen Endes (14) des Lichtfaserbündels (11) der Lichtquelle (20) entspricht, insbesondere gleich gewählt ist.

4. Endoskop, Exoskop oder Mikroskop nach einem der vorstehenden Ansprüche,**dadurch gekennzeichnet**, dassdie Form und/oder die Größe eines Faserendes am proximalen Ende (14) des Lichtfaserbündels (11) der Form und/oder der Größe der zugeordneten Einzellichtquellen (21) entspricht, insbesondere gleich gewählt ist.

5. Endoskop, Exoskop oder Mikroskop nach einem der vorstehenden Ansprüche,**dadurch gekennzeichnet**, dassEinzellichtquellen (21) Licht direkt in eine einzige oder einzelne Fasern (16) einkoppeln.

6. Endoskop, Exoskop oder Mikroskop nach einem der vorstehenden Ansprüche,**dadurch gekennzeichnet**, dasseine oder mehrere Optiken zwischen Einzellichtquellen (21) und dem proximalen Ende (14) des Lichtfaserbündels (11) angeordnet sind.

7. Endoskop, Exoskop oder Mikroskop nach einem der vorstehenden Ansprüche,**dadurch gekennzeichnet**, dassdas proximale Ende (14) des Lichtfaserbündels (11) mit der Lichtquelle (20) positionsfest insbesondere durch Verklebung verbunden ist.

8. Endoskop, Exoskop oder Mikroskop nach einem der vorstehenden Ansprüche,**dadurch gekennzeichnet**, dassdie Lichtquelle (20) im Endoskop (10), Exoskop oder Mikroskop, insbesondere im Griff des Endoskops (10), Exoskops oder Mikroskops angeordnet ist.

9. Endoskop, Exoskop oder Mikroskop nach einem der vorstehenden Ansprüche,**dadurch gekennzeichnet**, dassdie Lichtquelle (20) vom Endoskop (10), Exoskop oder Mikroskop abgesetzt ausgebildet ist und über ein Lichtfaserkabel zur Zuführung von Licht verbunden ist.

10. Endoskop, Exoskop oder Mikroskop nach Anspruch 9,
**dadurch gekennzeichnet**, dassdie Zuordnungsfunktion (30) abhängig von der abgesetzten Lichtquelle (20) wählbar ausgebildet ist.

11. Endoskop, Exoskop oder Mikroskop nach einem der vorstehenden Ansprüche insbesondere Anspruch 9 oder 10,
**dadurch gekennzeichnet**, dassMittel zur eindeutigen Festlegung der Position der Lichtquelle (20) gegenüber dem proximalen Ende (14) des Lichtfaserbündel (11) vorgesehen sind.

12. Endoskop, Exoskop oder Mikroskop nach einem der vorstehenden Ansprüche,**dadurch gekennzeichnet**, dassdie Teilbündel (13) am distalen Ende (1) des Endoskops (10) in unterschiedliche Richtungen weisen.

13. Endoskop, Exoskop oder Mikroskop nach einem der vorstehenden Ansprüche,**dadurch gekennzeichnet**, Steuereinheit (30) zum Ansteuern der Lichtquelle (20) zusätzlich geeignet ist, die Lichtquelle mit einem vorgegebenen Beleuchtungsprofil anzusteuern.

14. Verfahren zur Bestimmung einer Zuordnungsfunktion für ein Endoskop, Exoskop oder Mikroskop nach einem der vorstehenden Ansprüche,
**dadurch gekennzeichnet, dass** selektiv ein Teil der Einzellichtquellen (21) aktiviert werden und mit Hilfe eines optischen Detektors (40) erfasst wird, welches Teilbündel (13) am distalen Ende (15) des Lichtfaserbündels (11) Licht emittiert, und diese Zuordnung als Zuordnungsfunktion (30) insbesondere als Zuordnungstabelle (31) erfasst und gespeichert wird.

## Claims

1. Endoscope (10), exoscope or microscope,
comprising an optical fibre bundle (11) for transmitting light from the proximal end (14) thereof to the distal end (15) thereof, said optical fibre bundle exhibiting a contiguous bundle (12) at the proximal end (14) and a plurality of partial bundles (13) at the distal end (15),
comprising a light source (20) for coupling light into the one proximal end (14) of the optical fibre bundle (11) and comprising a multiplicity of individual-light sources (21) arranged in an array-like manner,
and comprising a control unit (30) for actuating the light source (20),
where
the light source (20) is provided with a multiplicity of individually actuatable individual-light sources (21),
**characterized**
**in that** the in that control unit (30) is suitable for actuating the individual-light sources (21) using an assignment function (31), which represents the assignment of a proximal fibre end (14) to a partial bundle (13), and in that the proximal ends (14) of the fibres (16) which are assigned to one partial bundle (13) are arranged on a surface in such a way that proximal ends (14) of fibres (15) assigned to a different partial bundle (13) are arranged therebetween, in that the control unit actuates the individually actuatable individual-light sources of the light source by means of a predetermined assignment function, which represents the assignment of a proximal fibre end of the optical fibre bundle to a partial beam and in such a way that only the individual-light sources that are assigned to a certain desired partial beam are activated.

2. Endoscope, exoscope or microscope according to Claim 1,
**characterized in that** the light source (20) includes an array of LEDs, semiconductor lasers, optical waveguide fibre ends.

3. Endoscope, exoscope or microscope according to Claim 1 or 2,
**characterized in that** the form and/or the size of the proximal end (14) of the optical fibre bundle (11) corresponds to, in particular is selected to be the same as, the light source (20).

4. Endoscope, exoscope or microscope according to one of the preceding claims,
**characterized in that** the form and/or the size of one fibre end at the proximal end (14) of the optical fibre bundle (11) corresponds to, in particular is selected to be the same as, the form and/or the size of the assigned individual-light sources (21).

5. Endoscope, exoscope or microscope according to one of the preceding claims,
**characterized in that** individual-light sources (21) couple light directly into a single or individual fibres (16).

6. Endoscope, exoscope or microscope according to one of the preceding claims,
**characterized in that** one or more optical units are arranged between individual-light sources (21) and the proximal end (14) of the optical fibre bundle (11).

7. Endoscope, exoscope or microscope according to one of the preceding claims,
**characterized in that** the proximal end (14) of the optical fibre bundle (11) is connected to the light source (20) in a positionally secured manner, in particular by means of adhesive bonding.

8. Endoscope, exoscope or microscope according to one of the preceding claims,
**characterized in that** the light source (20) is arranged in the endoscope (10), exoscope or microscope, in particular in the handle of the endoscope (10), exoscope or microscope.

9. Endoscope, exoscope or microscope according to one of the preceding claims,
**characterized in that** the light source (20) is embodied in a manner detached from the endoscope (10), exoscope or microscope and connected by means of an optical fibre cable for supplying light.

10. Endoscope, exoscope or microscope according to Claim 9,
**characterized in that** the assignment function (30) is configured in a manner selectable as a function of the detached light source (20).

11. Endoscope, exoscope or microscope according to one of the preceding claims, in particular Claim 9 or 10,
**characterized in that** means are provided for unambiguously setting the position of the light source (20) in relation to the proximal end (14) of the optical fibre bundle (11).

12. Endoscope, exoscope or microscope according to one of the preceding claims,
**characterized in that** the partial bundles (13) at the distal end (1) of the endoscope (10) point in different directions.

13. Endoscope, exoscope or microscope according to one of the preceding claims,
**characterized in that** control unit (30) for actuating the light source (20) is additionally suitable for actuating the light source with a predetermined illumination profile.

14. Method for determining an assignment function for an endoscope, exoscope or microscope according to one of the preceding claims,
**characterized in that** some of the individual-light sources (21) are activated selectively and an optical detector (40) is used to log which partial bundle (13) at the distal end (15) of the optical fibre bundle (11) emits light and this assignment is logged and stored as an assignment function (30), in particular as an assignment table (31).

## Revendications

1. Endoscope (10), exoscope ou microscope, comprenant un faisceau de fibres optiques (11) destiné à transmettre de la lumière depuis son extrémité proximale (14) jusqu'à son extrémité distale (15), lequel présente un faisceau cohérent (12) à l'extrémité proximale (14) et présente plusieurs faisceaux partiels (13) à l'extrémité distale (15),
comprenant une source de lumière (20) destinée à injecter de la lumière dans ladite extrémité proximale (14) du faisceau de fibres optiques (11), dotée d'une pluralité de sources de lumière individuelles (21) disposées à la manière d'une matrice et dotée d'une unité de commande (30) destinée à exciter la source de lumière (20),
la source de lumière (20) étant pourvue d'une pluralité de sources de lumière individuelles (21) excitables individuellement,
**caractérisé en ce**
**que** l'unité de commande (30) est conçue pour exciter les sources de lumière individuelles (21) en utilisant une fonction d'association (31), laquelle représente l'association d'une extrémité de fibre proximale (14) à un faisceau partiel (13), et **en ce que** les extrémités proximales (14) des fibres (16) qui sont associées à un faisceau partiel (13) sont disposées sur une surface de telle sorte qu'entre celles-ci sont disposées les extrémités proximales (14) des fibres (16) qui sont associées à un autre faisceau partiel (13), **en ce que** l'unité de commande, au moyen d'une fonction d'association prédéfinie qui représente l'association d'une extrémité proximale de fibre du faisceau de fibres optiques à un faisceau partiel, et excite les sources de lumière individuelles excitables individuellement de la source de lumière de telle sorte que seules sont activées les sources de lumière individuelles qui sont associées à un faisceau partiel souhaité donné.

2. Endoscope, exoscope ou microscope selon la revendication 1, **caractérisé en ce que** la source de lumière (20) possède une matrice de LED, de lasers en semiconducteur, d'extrémités de fibres optiques.

3. Endoscope, exoscope ou microscope selon la revendication 1 ou 2, **caractérisé en ce que** la forme et/ou la taille de l'extrémité proximale (14) du faisceau de fibres optiques (11) correspond à la source de lumière (20), est notamment choisie identique.

4. Endoscope, exoscope ou microscope selon l'une des revendications précédentes, **caractérisé en ce que** la forme et/ou la taille d'une extrémité de fibre à l'extrémité proximale (14) du faisceau de fibres optiques (11) correspond à la forme et/ou la taille des sources de lumière individuelles (21) associées, est notamment choisie identique.

5. Endoscope, exoscope ou microscope selon l'une des revendications précédentes, **caractérisé en ce que** les sources de lumière individuelles (21) injectent de la lumière directement dans une fibre unique ou dans des fibres (16) individuelles.

6. Endoscope, exoscope ou microscope selon l'une des revendications précédentes, **caractérisé en ce qu'**une ou plusieurs optiques sont disposées entre les sources de lumière individuelles (21) et l'extrémité proximale (14) du faisceau de fibres optiques (11).

7. Endoscope, exoscope ou microscope selon l'une des revendications précédentes, **caractérisé en ce que** l'extrémité proximale (14) du faisceau de fibres optiques (11) est reliée en position fixe à la source de lumière (20), notamment par collage.

8. Endoscope, exoscope ou microscope selon l'une des revendications précédentes, **caractérisé en ce que** la source de lumière (20) est disposée dans l'endoscope (10), l'exoscope ou le microscope, notamment dans la poignée de l'endoscope (10), de l'exoscope ou du microscope.

9. Endoscope, exoscope ou microscope selon l'une des revendications précédentes, **caractérisé en ce que** la source de lumière (20) est configurée décalée de l'endoscope (10), de l'exoscope ou du microscope et est reliée par le biais d'un câble à fibres optiques servant à l'acheminement de lumière.

10. Endoscope, exoscope ou microscope selon la revendication 9, **caractérisé en ce que** la fonction d'association (30) est réalisée sélectionnable en fonction de la source de lumière (20) décalée.

11. Endoscope, exoscope ou microscope selon l'une des revendications précédentes, en particulier la revendication 9 ou 10, **caractérisé en ce que** des moyens servant à définir explicitement la position de la source de lumière (20) par rapport à l'extrémité proximale (14) du faisceau de fibres optiques (11) sont présents.

12. Endoscope, exoscope ou microscope selon l'une des revendications précédentes, **caractérisé en ce que** les faisceaux partiels (13) sont orientés dans des directions différentes à l'extrémité distale (1) de l'endoscope (10).

13. Endoscope, exoscope ou microscope selon l'une des revendications précédentes, caractérisé en ce unité de commande (30) destinée à exciter la source de lumière (20) est en outre conçue pour exciter la source de lumière avec un profil d'éclairage prédéfini.

14. Procédé pour déterminer une fonction d'association pour un endoscope, un exoscope ou un microscope selon l'une des revendications précédentes, **caractérisé en ce qu'**une partie des sources de lumière individuelles (21) sont activées sélectivement et un détecteur optique (40) est utilisé pour détecter quel faisceau partiel (13) à l'extrémité distale (15) du faisceau de fibres optiques (11) émet de la lumière, et cette association est détectée et mémorisée en tant que fonction d'association (30), notamment en tant que tableau d'association (31).
